# EUROPEAN PATENT SPECIFICATION

(11) **EP 3 385 385 B1**
(45) Date of publication and mention of the grant of the patent: **11.01.2023**
(21) Application number: 16869848.8
(22) Date of filing: 01.11.2016
(51) Int. Cl.: C12N 15/29, C07K 14/415, A01H 5/00

(54) **USE OF TOBACCO GENE NTTCTP IN PLANTS AGAINST POTATO VIRUS Y**
VERWENDUNG VON TABAK-GEN-NTTCTP BEI PFLANZEN GEGEN KARTOFFELVIRUS Y
UTILISATION DU GÈNE NTTCTP DU TABAC CHEZ LES PLANTS POUR LUTTER CONTRE LE VIRUS Y DE LA POMME DE TERRE

(30) Priority: 01.12.2015 CN 201510858859
(43) Date of publication of application: 10.10.2018
(73) Proprietor: Guizhou Institute of Tobacco Science, Guiyang, Guizhou 550083 (CN)
(72) Inventor: GUO, Yushuang, Guiyang Guizhou 550083 (CN); JIA, Mengao, Guiyang Guizhou 550083 (CN); XIA, Fanjiang, Guiyang Guizhou 550081 (CN); ZHANG, Jinsong, Beijing 100101 (CN); CHEN, Shouyi, Beijing 100101 (CN); REN, Xueliang, Guiyang Guizhou 550083 (CN); ZHAO, Jiehong, Guiyang Guizhou 550083 (CN)
(74) Representative: EP&C
(86) International application number: PCT/CN2016/104255
(87) International publication number: WO 2017/092538

(56) References cited:
- CN-A- 103 374 059
- CN-A- 105 586 346
- US-B2- 6 545 202
- US-B2- 6 946 294
- MEENU GUPTA ET AL: "A Translationally Controlled Tumor Protein Negatively Regulates the Hypersensitive Response in Nicotiana benthamiana", PLANT AND CELL PHYSIOLOGY, vol. 54, no. 8, 22 July 2013 (2013-07-22), pages 1403-1414, XP55563273, UK ISSN: 0032-0781, DOI: 10.1093/pcp/pct090
- F. BRIOUDES ET AL: "Translationally controlled tumor protein is a conserved mitotic growth integrator in animals and plants", PROCEEDINGS OF THE NATIONAL ACADEMY OF SCIENCES OF THE UNITED STATES OF AMERICA, vol. 107, no. 37, 24 August 2010 (2010-08-24), pages 16384-16389, XP55563266, US ISSN: 0027-8424, DOI: 10.1073/pnas.1007926107
- F. BRIOUDES ET AL: "Translationally controlled tumor protein is a conserved mitotic growth integrator in animals and plants", PROCEEDINGS OF THE NATIONAL ACADEMY OF SCIENCES OF THE UNITED STATES OF AMERICA, vol. 107, no. 37, 24 August 2010 (2010-08-24), pages 16384-16389, XP55563429, US ISSN: 0027-8424, DOI: 10.1073/pnas.1007926107
- POLIANE, A.Z. ET AL.: 'Genome-Wide Analysis of Differentially Expressed Genes During the Early Stages of Tomato Infection by a Potyvirus' MOLECULAR PLANT-MICROBE INTERACTION S vol. 22, no. 3, 31 December 2009, ISSN 0894-0282 pages 352 - 361, XP055388062

## Description

### Technical Field

The invention belongs to the field of plant genetic engineering, and specifically, relates to the use of tobacco gene NtTCTP for resisting potato virus Y in plants.

### Background Art

Translationally controlled tumor protein (TCTP), also called p21 or p23, is a protein commonly expressed and distributed in eukaryotes. It was first discovered in mammalian tumor cells and regulated at the post-transcriptional level.

Based on a large number of studies in various organisms, it has been found that TCTP is involved in various cytological processes such as promotion of histamine release, apoptosis, microtubule assembly, ion balance, etc., and interacts with a series of proteins such as Polo kinase, tubulin, actin and sodium Potassium ATPase. In animals, TCTP participates in cell growth and differentiation, malignant transformation, stress resistance, and apoptosis and the like. Mice with TCTP gene knocked out showed embryonic lethality due to the lack of cell differentiation and excessive apoptosis. In drosophila, when the expression of TCTP in a specific organ is disrupted, the number of cells in the organ is decreased and defects in cell growth occur, eventually leading to decrease in size of the organ. TCTP mRNA expression in plants is closely related to mitosis, and darkness can induce TCTP mRNA accumulation in morning glory. In addition, TCTP proteins play an important role in regulating the long-distance transport of phloem proteins and the growth of pollen tubes. It has been found from recent studies that TCTP, as a positive regulatory factor of mitotic growth, can specifically regulate the cell cycle time in Arabidopsis. TCTP regulates cell differentiation through the TOR (Target of rapamycin) growth regulatory pathway. From the studies on cabbage, it has been found that TCTP regulates growth and development, and meanwhile is induced by the external environment. These existing studies have shown that, TCTP not only can regulate the growth and development of organisms, but also has exclusive functions in plants. Therefore, the study on this class of genes is of great significance in the study on resistance to PVY.

It is a key in plant antiviral breeding to dig into the gene against potato virus Y (PVY) in the plant genome.

### Summary of the Invention

The object of the present invention is to provide the use of tobacco gene NtTCTP for resisting potato virus Y in plants.

In order to achieve the purpose of the present invention, the tobacco gene NtTCTP according to the present invention, whose activity is closely related to the PVY-resistance of tobacco, has a gene sequence of:
i) a nucleotide sequence represented by SEQ ID NO: 1; or
ii) a nucleotide sequence represented by SEQ ID NO: 1 with one or more nucleotides substituted, deleted, and/or added therein, which expresses the same functional protein; or
iii) a nucleotide sequence that hybridizes to the sequence represented by SEQ ID NO: 1 under stringent conditions and expresses the same functional protein, the stringent conditions are given as follows: hybridizing in 0.1 × SSPE with 0.1% SDS or 0.1 × SSC solution with 0.1% SDS at 65 °C, and washing the membrane with the solution; or
iv) a nucleotide sequence having 90% or more homology with the nucleotide sequence of i), ii) or iii) and expressing the same functional protein.

The present invention also provides a protein encoded by the gene NtTCTP, and its amino acid sequence is represented by SEQ ID NO: 2.

The present invention also provides the use of the gene NtTCTP for resisting potato virus Y (PVY) in tobacco. That is, the tobacco gene NtTCTP is used to regulate the PVY-resistance of tobacco. The potato virus Y includes a PVY necrosis strain (N strain).

The use is utilizing RNAi technology to silence the gene NtTCTP in tobacco. For example, a nucleotide sequence represented by SEQ ID NO: 3 can be cloned into a plant expression vector, and then the recombinant expression vector is transformed into tobacco via Agrobacterium-mediated transformation, and screening is performed to obtain transgenic tobacco plants. Preferably the plant expression vector is pBin438.

The invention also provides a method for constructing a transgenic tobacco plant. An
can regulate the growth and development of organisms, but also has exclusive functions in plants. Therefore, the study on this class of genes is of great significance in the study on resistance to PVY.

It is a key in plant antiviral breeding to dig into the gene against potato virus Y (PVY) in the plant genome.

### Summary of the Invention

The object of the present invention is to provide the use of tobacco gene NtTCTP for resisting potato virus Y in tobacco as defined in the appended claims.

In order to achieve the purpose of the present invention, the tobacco gene NtTCTP according to the present invention, whose activity is closely related to the PVY-resistance of tobacco, has a gene sequence of a nucleotide sequence represented by SEQ ID NO: 1.

The present disclosure further describes (not part of the claimed invention)
i) a nucleotide sequence represented by SEQ ID NO: 1 with one or more nucleotides substituted, deleted, and/or added therein, which expresses the same functional protein; or
ii) a nucleotide sequence that hybridizes to the sequence represented by SEQ ID NO: 1 under stringent conditions and expresses the same functional protein, the stringent conditions are given as follows: hybridizing in 0.1 × SSPE with 0.1% SDS or 0.1 × SSC solution with 0.1% SDS at 65 °C, and washing the membrane with the solution; or
iii) a nucleotide sequence having 90% or more homology with the nucleotide sequence of SEQ ID NO: 1, i) or ii) and expressing the same functional protein.

The present disclosure further describes (not part of the claimed invention) a protein encoded by the gene NtTCTP, and its amino acid sequence is represented by SEQ ID NO: 2. The present disclosure further describes (not part of the claimed invention) the use of the gene NtTCTP for resisting potato virus Y (PVY) in tobacco. That is, the tobacco gene NtTCTP is used to regulate the PVY-resistance of tobacco. The potato virus Y includes a PVY necrosis strain (N strain).The use is utilizing RNAi technology to silence the gene NtTCTP in tobacco. For example, a nucleotide sequence represented by SEQ ID NO: 3 can be cloned into a plant expression vector, and then the recombinant expression vector is transformed into tobacco via Agrobacterium-mediated transformation, and screening is performed to obtain transgenic tobacco plants. Preferably the plant expression vector is pBin438.

The present disclosure further describes (not part of the claimed invention) a method for constructing a transgenic tobacco plant. An RNAi expression vector of gene NtTCTP is firstly constructed, and then the recombinant expression vector is transformed into tobacco via Agrobacterium-mediated transformation, and screening is performed to obtain transgenic tobacco plants. Preferably the starting vector is pBin438.

The present disclosure further describes (not part of the claimed invention) the use of the gene NtTCTP in the improvement of tobacco varieties. The invention also provides the use of the gene NtTCTP in breeding of PVY-resistant tobacco.

The present disclosure further describes (not part of the claimed invention) an expression vector carrying an expression cassette that inhibits the tobacco gene NtTCTP. Preferably, the expression vector carries the nucleotide sequence represented by SEQ ID NO: 3.

The present invention obtains the translationally controlled tumor protein gene NtTCTP by cloning from tobacco, and the gene has a full length of 507 bp, and encodes a protein containing 168 amino acids; and the present invention studies the role of the gene in the process of resisting PVY in tobacco. The gene is silenced in the cultivated tobacco, and the tobacco shows stronger resistance to the PVY, which can reach immune levels. The gene is overexpressed in the cultivated tobacco, and tobacco becomes more sensitive to the PVY. It is demonstrated that the gene plays an important role in the process of resisting PVY in tobacco and can be used in the breeding of PVY-resistant plants such as tobacco.

The RNAi expression vector of gene NtTCTP is constructed in the present invention, and the tobacco is genetically transformed through Agrobacterium-mediated transformation. The result shows that after the gene is silenced in tobacco, the resistance of tobacco against PVY can be increased by 50%-80%.

### Brief Description of the Drawings

FIG. 1 shows Western Blot verification results of TCTP genes in different tobacco strains in Example 3 of the present invention.
FIG. 2 shows leaf trait pictures of the gene TCTP-overexpressed strains O2 and O7, the gene TCTP-silenced strains Ri16 and Ri20, and wild-type tobacco, after PVY inoculation described in Example 3 of the present invention. It can be seen from the figures that, the veins of the overexpressed strains O2 and O7 are already necrotic, while the veins of the silenced strains Ri16 and Ri20 have no symptoms.

### Specific Modes for Carrying Out the Embodiments

The following examples are intended to illustrate the present invention Unless otherwise specified, the examples are carried out under conventional experimental conditions, such as those described in Sambrook J & Russell DW, Molecular Cloning: a Laboratory Manual (2001), or the conditions recommended in the manufacturer's instructions.

### Example 1: Cloning of Tobacco Gene NtTCTP

### I. Materials and methods

1. Experimental materials
   1.1 Cultivated tobacco variety "Shanxi Tobacco" was provided by the Breeding Engineering Center of Guizhou Institute of Tobacco Science.
   1.2 Reagents
      LA Taq DNA polymerase and restriction enzymes were purchased from TaKaRa. A DNA gel purification recovery kit was purchased from Tiangen Biochemical Technology (Beijing) Co., Ltd. T₄ DNA ligase was purchased from NEB; and pGEM-T vector was purchased from Promega Biotech Co., Ltd. ABI PRISMTM Optical 96-Well Reaction Plates and Optical Caps were purchased from ABI; *Escherichia coli* DH5α, *Agrobacterium tumefaciens* EHA105, and the plant expression vector pBin438 were all commercially available products. DNA synthesis and sequencing were completed by Huada Gene Technology Services Co., Ltd.
2. Experimental methods
   After logging in NCBI, primers for amplifying the full length gene were designed based on the full length sequence of the tobacco TCTP gene in Genbank (Accession NO: KM507327.1), and the restriction enzyme cutting sites BamH I and Sal I were added. The designed primer sequences were as follows:
      TCTPF1: 5'-GGATCCATGTTGGTTTATCAGGATCTTCTCT-3'
      TCTPR1: 5'-GTCGACTTAACACTTGACCTCCTTGAGTCCA-3'
   2.2 Full length amplification of the Tobacco Gene NtTCTP

The total RNA of "Shanxi tobacco" leaves was extracted, and subjected to reverse transcription to synthesize a first strand of cDNA. The following reagents were added into a 0.2 ml centrifuge tube to perform the PCR reaction:

| Reagent name | Volume |
|---|---|
| Tobacco cDNA | 1µl |
| TCTPF1 (10µM) | 1µl |
| TCTPR1 (10µM) | 1µl |
| 10×PCR buffer (Mg²⁺) | 2.5µl |
| dNTP Mixture (each 2.5mM) | 2µl |
| HiFi Taq DNA Polymerase (5U/µl) | 0.2µl |
| ddH₂O | 17.3µl |

The PCR reaction procedure was as follows: heating the lid, pre-denaturing at 95°C for 5 minutes; then performing 35 cycles of denaturing at 95°C for 30s, annealing at 55°C for 30s, and extending at 72°C for 30s.

The DNA fragment obtained by amplification was sequenced, with the result shown in SEQ ID NO: 1.

### Example 2: Construction of Tobacco Gene NtTCTP Transgenic strains

1. Construction of gene NtTCTP-overexpressed vectors
1.1 The plant expression vector pBin438 and the full length amplified PCR products were separately double-enzyme digested with Sal I and BamH I, and the enzyme digestion system was as follows:

| Reagent name | Volume |
|---|---|
| Sterile water | 7µL |
| 10×Buffer | 5µL |
| Sal I | 4µL |
| BamH I | 4µL |
| PCR recovery product/pBin438 | 30µL |

1.2 The enzyme digested products were detected by 1% agarose gel electrophoresis, and the enzyme digested PCR products and the large fragments of the vector pBin438 were recycled and ligated. The recycled products were directionally ligated in the following proportions. The ligation system was as follows:

| Reagent name | Volume |
|---|---|
| 10×T4 DNA Ligase Buffer | 1µL |
| enzyme-digested PCR product | 7µL |
| enzyme-digested Large DNA fragments of pBin438 | 1µL |
| T4 DNA Ligase | 1µL |

Ligation was performed at 16°C for 16 hours or longer, so as to improve the ligation efficiency.
1.3 Transformation of *Agrobacterium tumefaciens* with recombinant plasmid
1) 0.5 to 1.0 µL of DNA was taken, added into the competent cells of *Agrobacterium tumefaciens,* and gently mixed; the mixture was kept in ice bath for 5 min, then quickly put into liquid nitrogen and frozen for 5 min;
2) the mixture was thawed in a 37 °C water bath, and added with 800 µL YEB liquid medium, and then cultured under shaking at 28 °C and 100 r/min for 4 h;
3) the mixture was centrifuged for 5 minutes at 4000 r/min, and most supernatant was discarded with about 100 µL left to suspend the bacteria;
4) the suspended bacteria was coated on solid YEB medium containing 40 mg/L rifampicin (Rif), 50 mg/L streptomycin (Sm) and 100 mg/L kanamycin (Km), and cultured at 28°C for 48 to 96 h.

2. Construction of silencing vector
The forward sequence and the reverse complementary sequence of a 330 bp fragment from 1^{st} bp to 330^{th} bp of the tobacco gene NtTCTP was connected through an intermediate sequence, and the restriction enzyme cutting sites BamH I and Sal I were added. The designed sequence was synthesized by Sangon Biotech, Shanghai, and the synthesized sequence was represented by SEQ ID NO: 3. The synthesized fragment was digested with enzymes BamH I and Sal I, and ligated with the pBin438 vector which was digested in the same manner, so as to construct a final silencing vector.
*3. Agrobacterium tumefaciens-mediated* genetic transformation of tobacco
Tobacco leaf dishes were pre-cultured for 2-3 days, and then infected with *Agrobacterium tumefaciens* EHA105 containing the plant expression vector. After co-culture for 3 to 4 days, the leaf dishes were taken out and sterilized for 2 to 3 h in a liquid medium containing a bactericide, and then transferred to a screening medium for screening and differentiation culture. The resistant buds began to form via differentiation about 3 weeks later. When the resistant buds grew to a height of 3 to 4 cm, they were cut from the base of the explants and inoculated onto a rooting medium for rooting culture. After the root system of the plantlets was developed, acclimatizating and transplanting were performed. A large number of resistant plants that survived after transplanting were obtained. Plants that are positive in PCR detection were transplanted into large flower pots until the seeds were harvested.
Minimal medium: MS medium, pH 5.8, 0.8% agar.
Screening medium: minimal medium + 25 mg/L Km + 100 mg/L cephalosporin.
Differentiation medium: minimal medium + 1.0 mg/L 6-BA + 0.1 mg/L NAA, pH 5.8, 0.75% agar.
Rooting medium: MS medium + 0.5 mg/L NAA, pH 5.8, and 0.75% agar.
Transgenic materials, TCTP gene-overexpressed strains O2 and O7, and TCTP gene -silenced strains Ri16 and Ri20 were obtained.

### Example 3: study on PVY-resistance of transgenic tobacco

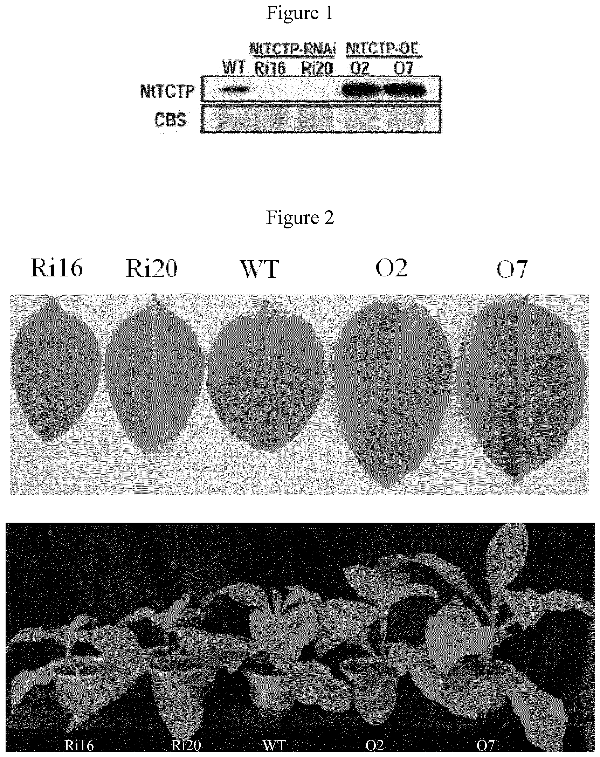

1. Materials and Methods
   1.1 Experimental Materials
      The gene NtTCTP overexpressed transgenic tobacco strains O2 and O7 constructed in Example 2.
      The gene NtTCTP-silenced transgenic tobacco strains Ri16 and Ri20 constructed in Example 3.
      The PVY strain was a PVY necrosis strain (N strain).
   1.2 Experimental methods
      1.2.1 Preparation of PVY inoculum: fresh diseased leaves were added to a crusher, and crushed together with a small amount of phosphate buffer solution; the diseased leaf residue was filtered off with gauze, and the fresh juice was taken to prepare the PVY inoculum.
      1.2.2 PVY inoculation: the inoculation period was the 4-leaf stage of the tobacco. 2 to 3 young leaves of the tobacco plant to be inoculated were selected and gently rubbed with sandpaper to generate light graze, and then the PVY inoculum was brushed on the rubbed leaves by a brush.
      1.2.3 Data investigation: the time at which the plant was most severely diseased was selected to investigate the incidence rate and the disease grade of PVY.
         The grade of disease was investigated according to the following standards:
         Grade 0: The whole plant was disease-free or had leaves mottled very slightly.
         Grade 1: 1/10 to 1/3 of the leaves showed slight necrosis of small veins, or stipple streak symptom and slight deformation occurred, the rest leaves of the whole plant were asymptomatic, and the plants had no obvious dwarfing.
         Grade 2: 1/3 to 1/2 of the leaves showed stipple streaks or vein necrosis, most of the small veins of the diseased leaves were necrotic, resulting in a large area of leaf yellowing and withering, and the plant dwarfed to 2/3 or more of the height of normal plants.
         Grade 3: 1/2 to 2/3 of the leaves showed relatively serious vein necrosis or more than 2/3 of the leaves showed stipple streak necrosis, single leaves were withered in a large area, and a few leaves fell off. Or the leaves of the whole plant began to turn yellow.
         Grade 4: the leaves of the whole plant showed serious vein necrosis or deformation and atrophy of leaves, about 2/3 of the leaves were wilted and sheded, the diseased plant dwarfed to 1/2 to 1/3 of the height of normal tobacco plants, or the whole plant was withered.
            Disease index DI = [Σ(number of diseased plants at each grade × the value of the disease grade) / (total number of investigated plants × the value of the highest grade)] × 100
2. Experimental results
   (1) Validation of the expression of NtTCTP gene in different tobacco strains
      Western Blot validation showed that, the TCTP gene was not expressed in the two silenced strains Ri16 and Ri20, indicating that the TCTP gene was successfully silenced, and the expression levels in 2 overexpressed strains O2 and O7 both exceeded that in the control (WT), indicating that the TCTP gene overexpression was also successfully constructed (Figure 1).
   (2) Identification results on disease resistance in the NtTCTP gene-overexpressed and gene-silenced strains

14 days after inoculation, the status of the disease was investigated. Both silenced strains Ri16 and Ri20 were immune, and showed no disease, with a disease index of 0, and the disease resistance was greatly improved. However, the overexpressed strains O2 and O7 showed necrosis more serious than the wild type control (WT), and the disease was more serious (Figure 2), wherein the disease index of the O2 strain was 96.97, the disease index of the O7 was 91.92, and the disease index of the control wild-type tobacco was 85.86.

The above results indicated that silencing TCTP gene in tobacco can enhance the resistance of plants against PVY, and the gene has important application value.

### Industrial applicability

The activity of the tobacco gene NtTCTP provided by the present invention is closely related to the resistance of tobacco against PVY. By construction of RNAi expression vector of the gene NtTCTP and genetic transformation via Agrobacterium-mediated transformation, it is shown that, after the gene is silenced in tobacco, the resistance of tobacco against PVY is enhanced, and the resistance can be increased by 50%-80%.

## Claims

1. Use of a tobacco gene NtTCTP for resisting potato virus Y in tobacco; wherein the tobacco gene NtTCTP is:
i) a nucleotide sequence represented by SEQ ID NO: 1;

2. The use according to claim 1, **characterized in that** the gene NtTCTP in tobacco is silenced using RNAi technology.

## Patentansprüche

1. Verwendung eines Tabakgens NtTCTP für Resistenz gegen das Kartoffelvirus Y in Tabak; wobei das Tabakgen NtTCTP:
i) eine Nukleotidsequenz, dargestellt durch SEQ ID NO: 1, ist.

2. Verwendung nach Anspruch 1, **dadurch gekennzeichnet, dass** das Gen NtTCTP in Tabak unter Verwendung von RNAi-Technologie stillgelegt wird.

## Revendications

1. Utilisation d'un gène de tabac NtTCTP pour résister au virus Y de la pomme de terre dans le tabac ; dans laquelle le gène de tabac NtTCTP est :
i) une séquence nucléotidique représentée par SEQ ID NO : 1.

2. Utilisation selon la revendication 1, **caractérisée en ce que** le gène NtTCTP dans le tabac est éteint en utilisant la technologie RNAi.
